# EUROPÄISCHE PATENTANMELDUNG

(11) **EP 3 001 177 A1**
(43) Veröffentlichungstag der Anmeldung: **30.03.2016**
(21) Anmeldenummer: 14186884.4
(22) Anmeldetag: 29.09.2014
(51) Int. Cl.: G01N 21/03, G01N 15/02, G01N 15/06, G01N 15/14

(54) **Vorrichtung zum Erfassen von Partikeln in einer Flüssigkeit**

(71) Anmelder: Grundfos Holding A/S, 8850 Bjerringbro (DK)
(72) Erfinder: Iversen, Kåre, 8870 Langå (DK); Mathis Dahlqvist,, 7100 Vejle (DK); Smith, Christian Guldbæk, 8541 Skødstrup (DK)
(74) Vertreter: Patentanwälte Vollmann & Hemmer

(57) **Zusammenfassung**

Die Vorrichtung dient zum qualitativen und quantitativen Erfassen von Partikeln in einer Flüssigkeit und weist eine Lichtquelle (1), einen optischen Sensor (2) und einen dazwischen angeordneten Probenträger (4) zur Aufnahme von zu untersuchender Flüssigkeit auf. Der Probenträger (4) ist relativ zumindest zum Sensor (2) bewegbar und über einen Flüssigkeitseinlass (9) mit einer Leitung (11) zum Zuführen von Flüssigkeit und über einen Flüssigkeitsauslass (10) mit einer Leitung (12) zum Abführen von Flüssigkeit verbindbar. Der Probenträger (4) ist austauschbar in einer Aufnahme der Vorrichtung angeordnet, so dass bei Verschmutzung des Probenträgers (4) dieser durch einen anderen schnell und einfach ersetzt werden kann.

## Beschreibung

Die Erfindung betrifft eine Vorrichtung zum qualitativen und/oder quantitativen Erfassen von Partikeln in einer Flüssigkeit mit den im Oberbegriff des Anspruchs 1 angegebenen Merkmalen, ein Verfahren zum Betrieb einer solchen Vorrichtung sowie einen Probenträger für eine solche Vorrichtung.

Eine gattungsgemäße Vorrichtung ist aus EP 2 469 264 A1 bekannt. Sie weist eine Lichtquelle und einen optischen Sensor auf, zwischen denen ein Probenträger zur Aufnahme von zu untersuchender Flüssigkeit angeordnet ist, der relativ zum Sensor bewegbar ist und der ein Fenster aufweist, in dem die zu untersuchende Flüssigkeit angeordnet ist, welches von der Lichtquelle durchstrahlt wird, so dass in der Flüssigkeit innerhalb des Fensters befindliche Partikel mittels des Sensors erfassbar sind. Durch Relativbewegung zwischen Probenträger und der optischen Anordnung können eine Vielzahl von Abschnitten des Fensters und der darin befindlichen Flüssigkeit erfasst werden, so dass nach Auswertung in der dort beschriebenen Weise Art und Anzahl der innerhalb des Fensters in der Flüssigkeit befindlichen Partikel erfasst werden können. Zu dem Verfahren im Einzelnen wird neben der genannten Druckschrift noch auf WO 2010/063293 A1 sowie WO 2014/094790 A1 verwiesen.

Für Untersuchungen unterschiedlicher Flüssigkeitsproben ist der in EP 2 469 264 A1 nur schematisch dargestellte Probenträger in eine Zuführleitung und eine Abführleitung eingegliedert, die jeweils über Ventile fest mit dem Probenträger und der Vorrichtung verbunden sind und über die ein Flüssigkeitsaustausch im Probenträger erfolgen kann.

Eine solche Anordnung hat sich grundsätzlich bewehrt, bedarf jedoch insbesondere bei der kontinuierlichen Überwachung, wie sie zum Beispiel zur Überwachung von Trinkwasser erforderlich ist, einer regelmäßigen Wartung. Dabei ist der Probenträger zumindest im Bereich des Fensters nicht nur von außen, sondern auch von innen zu reinigen, wenn sich beispielsweise Partikel dort angelagert haben, sei es in Form eines Bakterienfilms, einer Veralgung oder dergleichen. Das Reinigen des Probenträgers ist aufwendig, zudem kann während dieser Zeit die Vorrichtung nicht eingesetzt werden.

Vor diesem Hintergrund liegt der Erfindung die Aufgabe zugrunde, eine gattungsgemäße Vorrichtung so auszubilden, dass mit geringem Wartungsaufwand zuverlässiger Langzeitbetrieb möglich ist. Im Weiteren soll ein entsprechend ausgebildeter Probenträger geschaffen werden sowie ein Verfahren zum Betrieb der Vorrichtung.

Diese Aufgabe wird gemäß der Erfindung durch eine Vorrichtung mit den in Anspruch 1 angegebenen Merkmalen gelöst, ein Probenträger hierfür ist in Anspruch 12 angegeben, ein Verfahren zum Betrieb der Vorrichtung in Anspruch 21. Vorteilhafte Ausgestaltungen der Erfindung sind in den Unteransprüchen, der nachfolgenden Beschreibung und der Zeichnung angegeben, wobei die in den Unteransprüchen und der Beschreibung angegebenen Merkmale jeweils für sich aber auch in geeigneter Kombination die Ansprüche 1, 12 und 21 weiter ausgestalten können.

Die erfindungsgemäße Vorrichtung zum qualitativen und/oder quantitativen Erfassen von Partikeln in einer Flüssigkeit weist eine Lichtquelle, einen optischen Sensor und einen dazwischen angeordneten Probenträger zur Aufnahme von zu untersuchender Flüssigkeit auf, wobei der Probenträger relativ zumindest zum Sensor bewegbar ist und über einen Flüssigkeitseinlass mit einer Leitung zum Zuführen von Flüssigkeit und über einen Flüssigkeitsauslass mit einer Leitung zum Abführen von Flüssigkeit verbindbar ist. Gemäß der Erfindung ist der Probenträger austauschbar in einer Aufnahme der Vorrichtung angeordnet.

Grundgedanke der vorliegenden Erfindung ist es, eine Vorrichtung, bei welcher der Probenträger selbst über Leitungen zum Austausch der darin befindlichen Flüssigkeit vorgesehen ist, so auszubilden, dass dieser austauschbar in der Vorrichtung angeordnet ist, hierfür also eine gesonderte Aufnahme vorzusehen, aus der bzw. in die der Probenträger aus- bzw. einführbar ist.

Dabei ist unter austauschbar im Sinne der Erfindung ein vorzugsweise werkzeugfreier Austausch zu verstehen. Es sind der Probenträger und die Aufnahme innerhalb der Vorrichtung vorteilhaft so angeordnet, dass diese von außen zugänglich sind, das heißt der Probenträger ohne Demontage von Vorrichtungsteilen ausgetauscht werden kann. Die erfindungsgemäße Lösung sieht somit einen austauschbaren Probenträger vor, obgleich der Austausch der Flüssigkeit typischerweise nicht durch Austausch des Probenträgers, sondern durch Flüssigkeitsaustausch innerhalb des Probenträgers erfolgt, nämlich über die Zuführleitung und die Abführleitung. Dies schließt in einer Weiterbildung der Erfindung allerdings nicht aus, dass in der Vorrichtung alternativ auch Probenträger Verwendung finden können, die zum Verbleib der Flüssigkeit im Probenträger vorgesehen und bestimmt sind.

Die erfindungsgemäße Lösung ist vorteilhaft für die Trinkwasseranalyse vorgesehen, kann jedoch grundsätzlich auch in allen anderen Bereichen eingesetzt werden, wo es auf die qualitative und/oder quantitative Erfassung von Partikeln in einer Flüssigkeit ankommt. Nur beispielhaft sei hier erwähnt, Kühlwasser, Prozesswasser, Abwasser, flüssige Chemikalien, Druckertinte und dergleichen. Dabei sind unter Partikeln im Sinne der vorliegenden Erfindung typischerweise Körper in einer Größenordnung im 100nm Bereich aufwärts zu verstehen, also beispielsweise Bakterien, organische oder anorganische Körper oder andere Teilchen. Die Erfindung ist grundsätzlich nicht auf die Partikelgröße beschränkt, eine natürliche Begrenzung ergibt sich allerdings durch die Wellenlänge der eingesetzten Lichtquelle und des detektierenden Sensors. Dabei ist unter Lichtquelle mit optischem Sensor jede geeignete elektromagnetische Quelle mit einem, für diesen Wellenlängenbereich empfindlichen Sensor, zu verstehen, die auch außerhalb des sichtbaren Lichtbereiches liegen können.

Der Probenträger ist so ausgebildet, dass zwischen den Leitungsanschlüssen im Probenträger ein geschlossener Kanal gebildet ist, der zumindest abschnittsweise an zwei vorzugsweise abgewandt gegenüberliegenden Seiten, zweckmäßigerweise der Ober- und der Unterseite, eine durchsichtige Wandung aufweist, so dass über einen Abschnitt des Kanals ein Fenster gebildet wird, welches von der Lichtquelle durchstrahlt und vom optischen Sensor erfasst werden kann. Grundsätzlich muss der Kanal nicht geschlossen sein, es ist denkbar, dass er beispielsweise an der Oberseite offen ist, um beispielsweise eine Entgasung der Flüssigkeit vor der Untersuchung zu ermöglichen. Der geschlossene Kanal hingegen ermöglicht eine Druckerhöhung während der Untersuchung, wodurch eine Entgasung und die damit verbundene Blasenbildung verhindert werden kann.

Um das Zu- und Abführen von Flüssigkeit in den Probenträger gezielt steuern zu können, sind innerhalb der Vorrichtung Absperrmittel vorgesehen. Dabei ist mindestens ein Absperrventil in der Zuführleitung vorzusehen, vorzugsweise jedoch ein weiteres in der Abführleitung, um den Kanal innerhalb des Probenträgers abschließen zu können, so dass eine Strömung im Kanal während der Untersuchung ausgeschlossen und somit eine Beruhigung der Flüssigkeit und der darin befindlichen Partikel sichergestellt werden.

Besonders vorteilhaft ist es, wenn beide Leitungen, also die Zuführ- und die Abführleitung von unten in den Probenträger münden, und zwar vorzugsweise in Einschubrichtung nebeneinander. Eine solche Anordnung ist vorteilhaft, da dann etwaige an den Anschlüssen am Probenträger anhaftende Flüssigkeitsreste beim Herausziehen des Probenträgers aus der Aufnahme nach unten ins Freie abtropfen können, ohne dass die Gefahr besteht, dass sich die Flüssigkeit in der Aufnahme sammelt oder außen auf dem Probenträger verteilt.

Vorteilhaft ist innerhalb der Vorrichtung die Lichtquelle auf einer Seite des Probenträgers und der optische Sensor auf der anderen Seite des Probenträgers angeordnet, und zwar vorzugsweise der optische Sensor oberhalb des Probenträgers und die Lichtquelle unterhalb. Dadurch werden auf natürliche Weise Ablagerungen auf dem Sensor vermieden. Da die Erfassung von Partikeln in der Flüssigkeit nur bei Stillstand der Flüssigkeit erfolgen kann, ist vorteilhaft innerhalb der Vorrichtung der Probenträger feststehend und die optische Vorrichtung, bestehend aus Lichtquelle, Sensor und gegebenenfalls weiteren optischen Bauteilen, bewegbar zum Probenträger angeordnet, und zwar vorzugsweise schrittweise, so dass eine Bewegung des Probenträgers und damit der darin befindlichen Flüssigkeit bzw. der darin befindlichen Partikel zwischen den einzelnen Erfassungsvorgängen vermieden werden kann.

Als besonders vorteilhaft hat es sich erwiesen, die optische Achse von Lichtquelle und optischem Sensor und gegebenenfalls weiteren optischen Komponenten in einen Winkel zwischen 4° und 8° zur senkrechten Durchstrahlungsrichtung durch den Probenträger anzuordnen und diese optischen Komponenten in dieser Stellung längs des Probenträgers schrittweise zu bewegen.

Gemäß einer bevorzugten Ausgestaltung ist die Vorrichtung so ausgebildet, dass Mittel zwischen Aufnahme und Probenträger vorgesehen sind, welche eine Leitungsverbindung zu dem Flüssigkeitseinlass und/oder zu dem Flüssigkeitsauslass nur zulassen, wenn der Probenträger in seiner bestimmungsgemäßen Position in der Aufnahme angeordnet ist. Bevorzugt sind die Mittel so ausgestaltet, dass sowohl die Leitungsverbindung zu dem Flüssigkeitsauslass als auch die Leitungsverbindung zu dem Flüssigkeitseinlass nur in dieser bestimmungsgemäßen Position gebildet werden können.

Dies kann vorteilhaft dadurch erreicht werden, dass ein erster Schalter vorgesehen ist, welcher beim Erreichen der bestimmungsgemäßen Position des Probenträgers in der Aufnahme schaltet. Dies kann ein Mikroschalter sein, der elektrisch arbeitet oder ein mechanischer Schalter, welcher die Ventilfreigabe steuert.

Um sicherzustellen, dass der Probenträger nur in der dafür vorgesehen Stellung in die Aufnahme einführbar ist, sind gemäß einer Weiterbildung der Erfindung Formschlussmittel zwischen Aufnahme und Probenträger vorgesehen. Wenn der Probenträger in die Aufnahme einschiebbar ist, so können solche Formschlussmittel in einem asymmetrischen Profil vom Probenträger und Aufnahme gebildet sein, oder durch beispielsweise endseitige Vorsprünge, die nur in einer Stellung in eine entsprechende aufnahmeseitige Ausnehmung eingeführt werden können oder in anderer geeigneter Weise.

Um sicherzustellen, dass der Probenträger auch während des Erfassens in seiner bestimmungsgemäßen Position in der Aufnahme verbleibt, ist gemäß einer Weiterbildung der Erfindung ein vorzugsweise schwenkbarer Riegel vorgesehen, mit welchem der Probenträger in seiner bestimmungsgemäßen Position in der Aufnahme formschlüssig feststellbar ist. Ein solcher Riegel kann nach Einschieben des Probenträgers in die Aufnahme eingeschwenkt werden und damit den Probenträger in der Aufnahme festsetzen. Dabei ist vorteilhaft ein zweiter Schalter vorgesehen, welcher die verriegelnde Stellung des Riegels detektiert, der ebenfalls in die Steuerung für die Leitungsverbindung zu dem Flüssigkeitseinlass und/oder dem Flüssigkeitsauslass geschaltet sein kann, um nicht nur sicherzustellen, dass eine Leitungsverbindung nur dann erfolgt, wenn der Probenträger in seiner bestimmungsgemäßen Position in der Aufnahme ist, sondern darüber hinaus nur dann, wenn dieser auch in dieser Position verriegelt ist. Auch dieser Schalter kann ein elektrischer Mikroschalter oder ein mechanischer Schalter sein, welcher mit der Ventilsteuerung gekoppelt ist.

Der Probenträger für die erfindungsgemäße Vorrichtung weist vorteilhaft einen Tragrahmen auf, der an zwei Seiten, vorzugsweise an der Ober- und der Unterseite (bezogen auf die bestimmungsgemäße Position in der Aufnahme), jeweils mit einer durchsichtigen Platte abgeschlossen ist. Ein solcher Probenträger kann kostengünstig aus Kunststoff gefertigt werden, wobei der Rahmen zum einen für einen stabilen Aufbau des Probenträgers sorgt, zum anderen den innerhalb des Probenträgers gebildeten Kanal zumindest seitlich begrenzen kann. Die durchsichtigen Platten können vorteilhaft ebenfalls aus Kunststoff bestehen und stoffschlüssig mit dem Rahmen verbunden sein.

Um zu verhindern, dass durchsichtige Platten beim Einschieben bzw. Herausziehen des Probenträgers in die bzw. aus der Aufnahme zerkratzt werden, sind diese vorteilhaft gegenüber dem umfänglich umlaufenden Rahmenteil zurückspringend angeordnet. Dabei ist das Rahmenteil vorteilhaft nach innen hin, also dort wo die durchsichtigen Platten angeordnet sind, abgesetzt ausgebildet, so dass die Platten jeweils in eine durch den Rahmen gebildete Vertiefung eingegliedert sind, welche tiefer als die Dicke einer Platte ist.

Vorteilhaft ist der Probenträger so ausgebildet, dass bei bestimmungsgemäßer Positionierung in der Aufnahme der Flüssigkeitseinlass und der Flüssigkeitsauslass an der Unterseite, und zwar vorzugsweise in Einschubrichtung nebeneinander angeordnet sind. Darüber hinaus sollten Flüssigkeitseinlass und Flüssigkeitsauslass möglichst nahe am Eingang der Aufnahme angeordnet sein, damit beim Herausziehen etwaige anhaftende Flüssigkeitstropfen nicht in der Vorrichtung verbleiben, sondern auf kürzestem Weg mit dem Probenträger herausgezogen werden. Das nebeneinander Anordnen hat den Vorteil, dass zuverlässig ausgeschlossen ist, dass Flüssigkeit vom Einlass mit Flüssigkeit vom Auslass in Kontakt kommt oder umgekehrt. Bei einer Anordnung der Anschlüsse nebeneinander ist es besonders zweckmäßig, zwischen dem Flüssigkeitseinlass und dem Flüssigkeitsauslass innerhalb des Probenträgers mindestens eine kanalbildende Rippe vorzusehen, die vorzugsweise einen Teil des Rahmens bildet. Es entsteht dann zwischen Flüssigkeitseinlass und Flüssigkeitsauslass ein Kanal, der einerseits durch den Rahmen und andererseits durch die Rippe begrenzt ist. Dieser Kanal ist am Ende des Probenträgers um 180° umgelenkt, läuft also zu beiden Seiten der Rippe. Durch diese Kanalführung, die praktisch zwei nebeneinander angeordnete endseitig miteinander verbundene Kanäle schafft, ist einerseits die Leitungsverbindung zwischen Einlass und Auslass sichergestellt, andererseits ein weiteres Untersuchungsfeld gebildet, das genutzt werden kann.

Um die bestimmungsgemäße Position des Probenträgers innerhalb der Aufnahme in einfacher Weise sicherzustellen, kann gemäß einer Weiterbildung der Erfindung an der Einsteckseite, also an der Seite, mit welcher der Probenträger in die Aufnahme eingeführt wird, ein Vorsprung vorgesehen sein, der außermittig angeordnet ist und ein Formschlussmittel zum Positionieren bildet, indem er in eine entsprechende Ausnehmung am Ende der Aufnahme eingreift. Alternativ können Probenträger und Aufnahme ein Querschnittsprofil aufweisen, welches ein Einschieben nur in der bestimmungsgemäßen Weise ermöglicht.

Der Rahmen des Probenträgers kann vorteilhaft als Kunststoffspritzgussteil ausgebildet werden, wobei die durchsichtigen Platten stoffschlüssig, das heißt durch Kleben oder Schweißen fest und dicht mit dem Rahmen verbunden werden. Dabei ist eine Schweißverbindung zu bevorzugen, dies setzt jedoch voraus, dass die Bauteile miteinander verschweißbar sind.

Um einen dichten Anschluss des Probenträgers an die innerhalb der Aufnahme der Vorrichtung befindlichen Leitungsanschlüsse zu gewährleisten, sind vorteilhaft Dichtmittel vorzusehen. Fertigungstechnisch besonders vorteilhaft ist es, wenn der Probenträger im Bereich der Ein- und Auslässe eine Ausnehmung, typischerweise eine flächige Vertiefung in dieser Seite des Rahmenteils aufweist, welche mit einem elastischen und dichtungsbildenden Kunststoff, beispielsweise Silikon, ausgefüllt ist. Dieser Kunststoff kann insbesondere im Bereich der Anschlüsse ringförmig überhöht ausgebildet sein, so dass sichergestellt ist, dass nach Einschieben des Probenträgers in die Aufnahme diese Erhöhungen dichtend um die entsprechenden Leitungsanschlüsse in der Aufnahme anliegen.

Um eine möglichst werkzeugfreie Handhabung des Probenträgers beim Einsetzen in die Aufnahme und Herausnehmen aus der Aufnahme zu gewährleisten, ist gemäß einer Weiterbildung der Erfindung ein Griffstück am Probenträger vorgesehen, welches vorzugsweise am Ende einer Schmalseite angeordnet ist und Teil des Rahmens bildet. Ein solches Griffstück kann zwischen Daumen und Finger gefasst und entsprechend manipuliert werden.

Besonders vorteilhaft ist es, wenn der Probenträger eine im Wesentlichen langgestreckte und flache Quaderform aufweist, wobei die Flachseiten mit den durchsichtigen Platten versehen sind und die langgestreckten Seiten in Einschubrichtung angeordnet sind. Hierbei kann der Probenträger stiftartig in die Aufnahme eingesetzt werden, ohne die Gefahr eines Verkantens, wobei die langgestreckte Form eine ausreichend lange Messstrecke innerhalb des Kanals gewährleistet.

Gemäß einer vorteilhaften Weiterbildung der Erfindung ist der geschlossene Kanal innenseitig, zumindest in dem Bereich, der zur optischen Erfassung der Flüssigkeit vorgesehen ist, und dort zumindest an der Innenseite der durchsichtigen Wandung mit einer Beschichtung und/oder einer Oberflächenstrukturierung versehen, welche die Bildung von Ablagerungen verhindert, vermindert oder zumindest verzögert. Die Beschichtung und/oder Oberflächenstrukturierung kann davon abhängen, für welche Flüssigkeiten der Probenträger vorgesehen ist. Bei Wasser beispielsweise ist einer Veralgung vorzubeugen aber auch anderen Ablagerungen, seien es Bakterien, Kalk oder dergleichen. Eine solche Beschichtung kann beispielsweise eine hydrophile oder hydrophobe sein. Die Oberflächen können Mikrostrukturen aufweisen, welche ein Anhaften von Ablagerungen erschweren. Es können auch strukturierte Oberflächen in Verbindung mit Beschichtungen vorgesehen sein. So können als Beschichtungen beispielsweise Acrylate, Silane oder Fluorpolymere und dergleichen verwendet werden, wie diese an sich bekannt sind.

Darüber hinaus kann die Vorrichtung selbst mit Mitteln zur Reinigung bestückt sein, sei es dass über eine Pumpe der Probenträger in Intervallen mit erhöhtem Druck und erhöhter Durchflussgeschwindigkeit gespült wird oder dass ein Ultraschallerreger vorgesehen ist, welcher insbesondere die durchsichtigen Wände des Probenträgers zum Zwecke der Reinigung beaufschlagt. Auch kann eine Reinigungseinrichtung vorgesehen sein, die beispielsweise den Probenträger in Intervallen mit einer Reinigungsflüssigkeit oder einem Reinigungsgas, wie beispielsweise Ozon, spült.

Vorteilhaft weist der Probenträger in dem Bereich, der zur optischen Erfassung der Flüssigkeit vorgesehen ist, mindestens eine Markierung auf, und zwar vorzugsweise zwischen den durchsichtigen Wandungen, insbesondere an einer Innenseite einer Wandung. Eine solche Markierung kann zur Kalibrierung der optischen Einrichtung genutzt werden, sie kann ferner dazu dienen, den Autofokus der optischen Einrichtung auf die gewünschte Ebene im Probenträger auszurichten. Auch können am Anfang und Ende des Messfensters Markierungen vorgesehen sein und damit den Bereich des Fensters markieren, der zur optischen Erfassung der Flüssigkeit vorgesehen ist. Eine solche Markierung kann durch Gravur oder Aufdruck auf eine der durchsichtigen Platten erfolgen. Vorteilhaft ist die Markierung sensornah angebracht, also auf der Innenseite der durchsichtigen Platte, die dem Sensor am nächsten ist.

Probenträger und Aufnahme können in weiten Bereichen hinsichtlich Abmessungen und Proportionen gestaltet werden, was sicherlich auch abhängig von der zu untersuchenden Flüssigkeit ist. Wenn Abwässer oder dergleichen zu untersuchen sind, werden größere Kanäle erforderlich sein als bei der Brauchwasserprüfung. Allerdings hat es sich in einer Vielzahl der Fälle als vorteilhaft erwiesen, wenn der geschlossene Kanal im Probenträger in dem Bereich, der zur optischen Erfassung der Flüssigkeit vorgesehen ist, einen vorzugsweise etwa rechteckigen Konolquer-schnitt aufweist, dessen Breite größer als dessen Höhe in senkrechter Durchstrahlungsrichtung ist. Dabei sollte das Verhältnis von Breite zu Höhe vorzugsweise 1,5 bis 3 betragen, also der Kanalquerschnitt 1,5- bis 3-mal so breit sein wie er hoch ist. So kann beispielsweise für die Trinkwasserprüfung die Kanalbreite 3 mm und die Kanalhöhe 1,5 mm betragen. Je flacher der Kanal ist, desto geringer sind die Reflektionen innerhalb des Kanals, insbesondere innerhalb der Flüssigkeit. Mit wachsender Kanalhöhe steigt zumindest, wenn die Beleuchtung, was bevorzugt ist, von unten erfolgt und der Sensor oben angeordnet ist, die thermisch bedingte Bewegung innerhalb der Flüssigkeit im Probenträger, da die Temperaturdifferenz zwischen der Unterseite und der Oberseite des Probenträgers größer wird. Dies ist jedoch unerwünscht, da die Flüssigkeit bei der Erfassung quasi stationär sein soll. Um Reflektionen innerhalb des Probenträgers gering zu halten, ist es von Vorteil, wenn der Rahmen des Probenträgers, also alle Bereiche, die nicht durchsichtig gestaltet sein müssen, eine möglichst lichtabsorbierende Oberfläche haben, beispielsweise schwarz sind.

Das erfindungsgemäße Verfahren zum Erfassen von Partikeln in einer Flüssigkeit mit der erfindungsgemäßen Vorrichtung sieht vor, dass zunächst der Probenträger in die bestimmungsgemäße Position in der Aufnahme der Vorrichtung verbracht wird, wonach in einem ersten Schritt über die Zuführleitung die zu untersuchende Flüssigkeit in den Probenträger eingeleitet wird, wonach in einem zweiten Schritt die Abführleitung abgesperrt wird und nach Druckaufbau innerhalb des Probenträgers in einem dritten Schritt die Zuführleitung verschlossen wird, wonach eine Verweilzeit lang gewartet wird und in einem vierten Schritt die optische Erfassung der Probe erfolgt. Dieses Verfahren ist besonders vorteilhaft, da durch den Druckaufbau innerhalb des Probenträgers eine Entgasung der Flüssigkeit verhindert, zumindest verlangsamt wird, so dass eine unerwünschte Blasenbildung unterdrückt wird. Die Verweilzeit dient dazu, die Partikel innerhalb der Flüssigkeit quasi stationär anzuordnen. Wenn Partikel wie beispielsweise Bakterien erfasst werden sollen, so genügt in der Regel eine Verweilzeit von etwa einer Minute um eine quasi stationäre Anordnung der Bakterien innerhalb der Flüssigkeit sicherzustellen. Sollen hingegen Partikel erfasst werden, wie sie durch Verunreinigungen wie Staub, Sand und dergleichen gebildet werden, ist die Verweilzeit gegebenenfalls länger zu wählen, insbesondere bei solchen Partikeln, deren spezifisches Gewicht deutlich größer oder kleiner als das der Trägerflüssigkeit ist. Hier wird die Verweilzeit so gewählt, dass die Partikel bis auf den Grund des Probenträgers absinken bzw. zur Oberseite aufsteigen können.

Da der erfindungsgemäße Probenträger typischerweise für mehrfachen Gebrauch ausgelegt ist, werden nach dem optischen Erfassen in einem fünften Schritt die Zuführleitung und die Abführleitung geöffnet, wonach die im Probenträger befindliche Flüssigkeit ersetzt, das heißt herausgespült wird und der Zyklus im zweiten Schritt beginnend, wiederholt wird. Üblicherweise kann der Probenträger über lange Zeit Verwendung finden. Erst wenn Ablagerungen oder andere Verunreinigungen das Erfassen der Partikel erschweren oder unmöglich machen, wird der Probenträger ersetzt, wobei dies durch Öffnen des Riegels und einfaches Herausziehen und Ersetzen durch einen neuen Probenträger und nachfolgendes Verschließen des Riegels erfolgen kann, also auch durch ungeschultes Personal.

Die Erfindung ist nachfolgend anhand eines in der Zeichnung dargestellten Ausführungsbeispiels näher erläutert. Es zeigen:
- Fig. 1: in schematischer Darstellung den Aufbau der erfindungsgemäßen Vorrichtung,
- Fig. 2: in schematischer Schnittdarstellung die Aufnahme der Vorrichtung mit darin eingeführtem Probenträger,

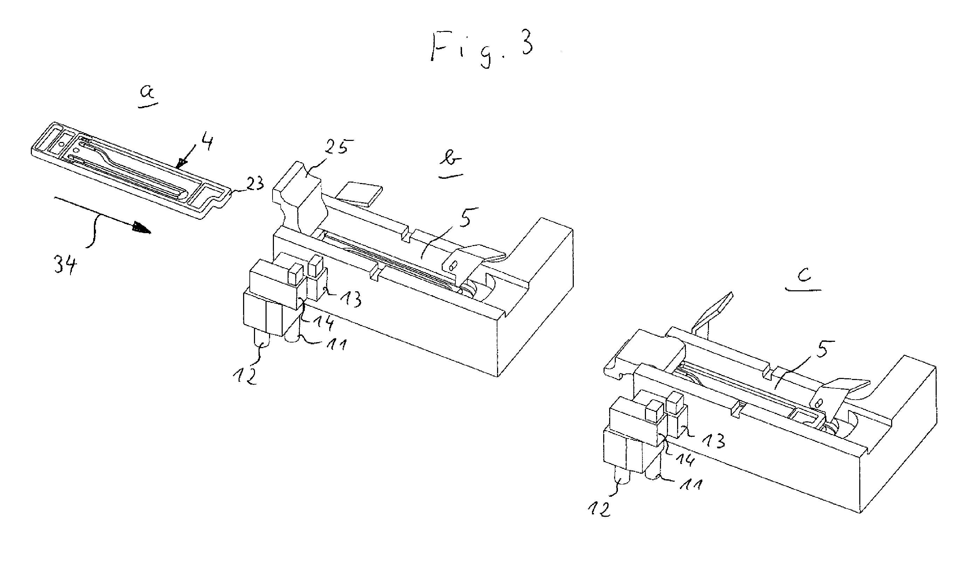

- Fig. 3: in vereinfachter perspektivischer Darstellung
a) den Probenträger
b) die Aufnahme und
c) die Aufnahme mit Probenträger,
- Fig. 4: eine Draufsicht auf den Probenträger,
- Fig. 5: eine Unteransicht des Probenträgers,

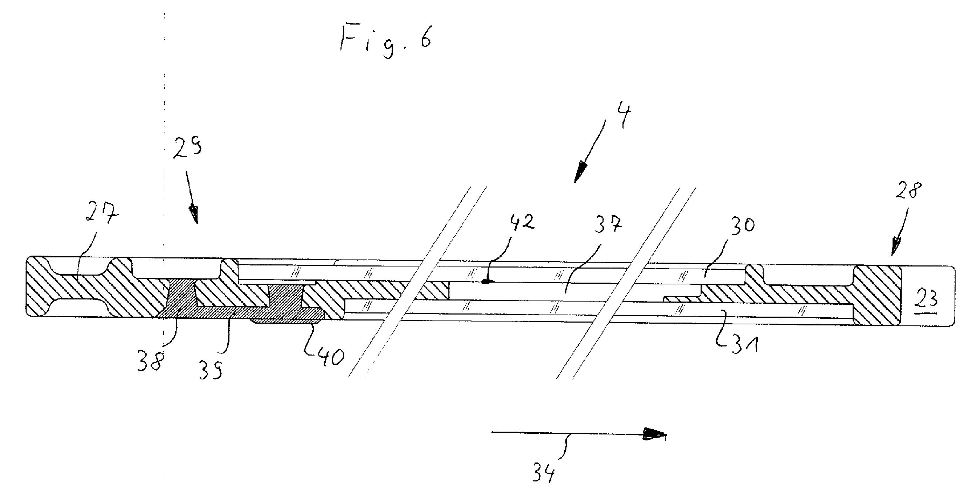

- Fig. 6: einen Schnitt längs der Schnittlinie VI- VI in Fig. 4 bzw. VI - VI in Fig. 5,

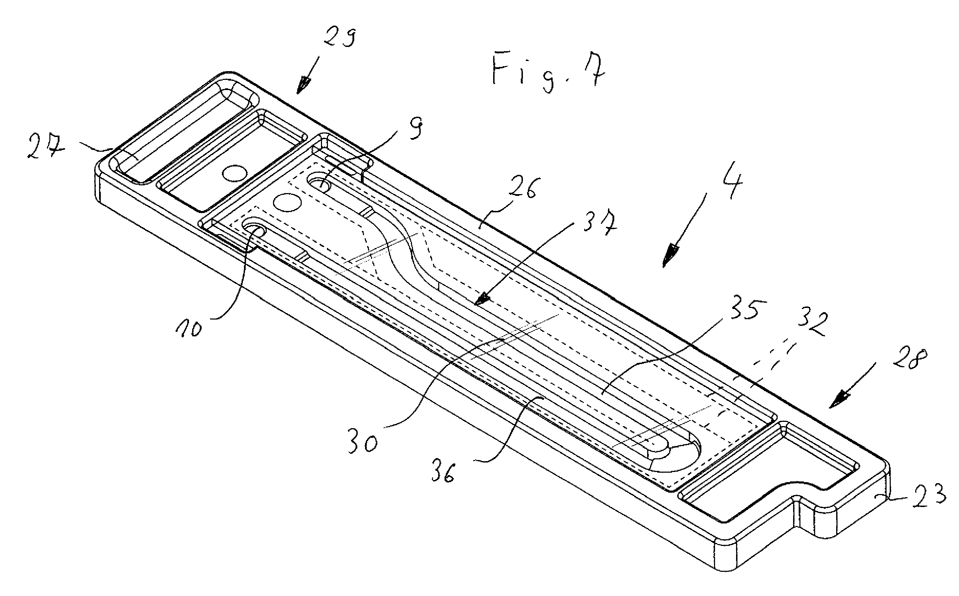

- Fig. 7: eine perspektivische Darstellung des Probenträgers von oben und

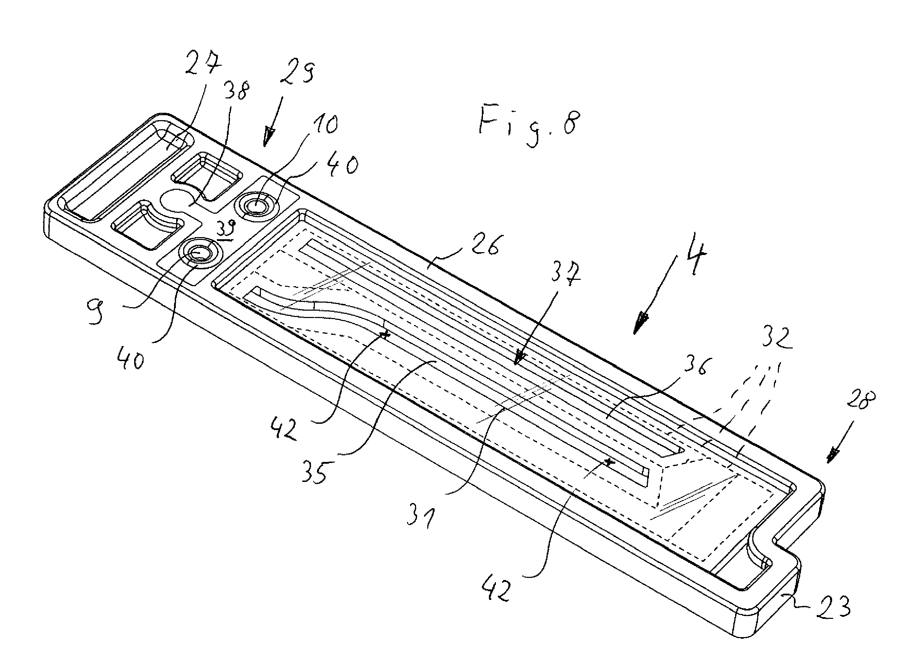

- Fig. 8: eine perspektivische Darstellung des Probenträgers von unten

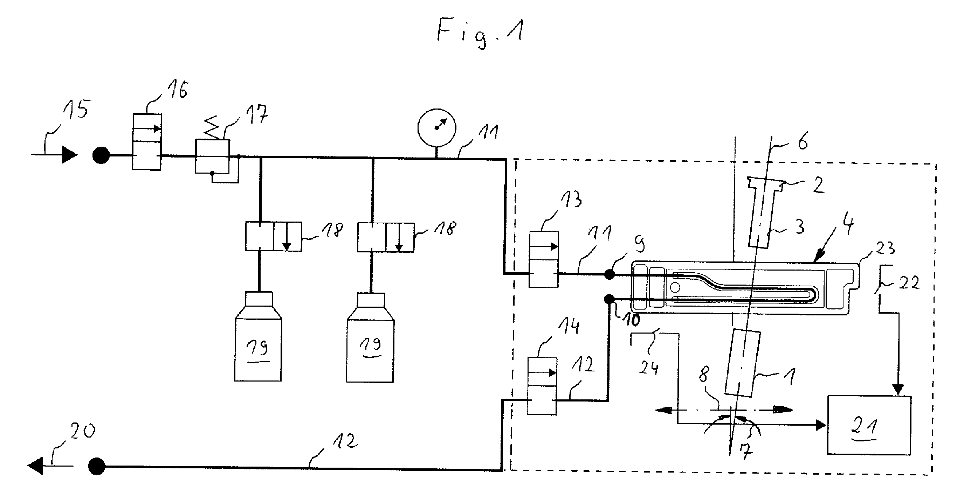

In Fig. 1 sind die wesentlichen Elemente der Vorrichtung zum qualitativen und quantitativen Erfassen von Partikeln in einer Flüssigkeit dargestellt, wobei der Kern der Vorrichtung durch den in unterbrochenen Linien dargestellten Rahmen gebildet ist und die übrigen Bauelemente zwar zur Vorrichtung gehören, jedoch zur Peripherie, so dass sie nicht notwendigerweise räumlich mit der Vorrichtung verbunden, das heißt in einem gemeinsamen Gehäuse angeordnet sein müssen.

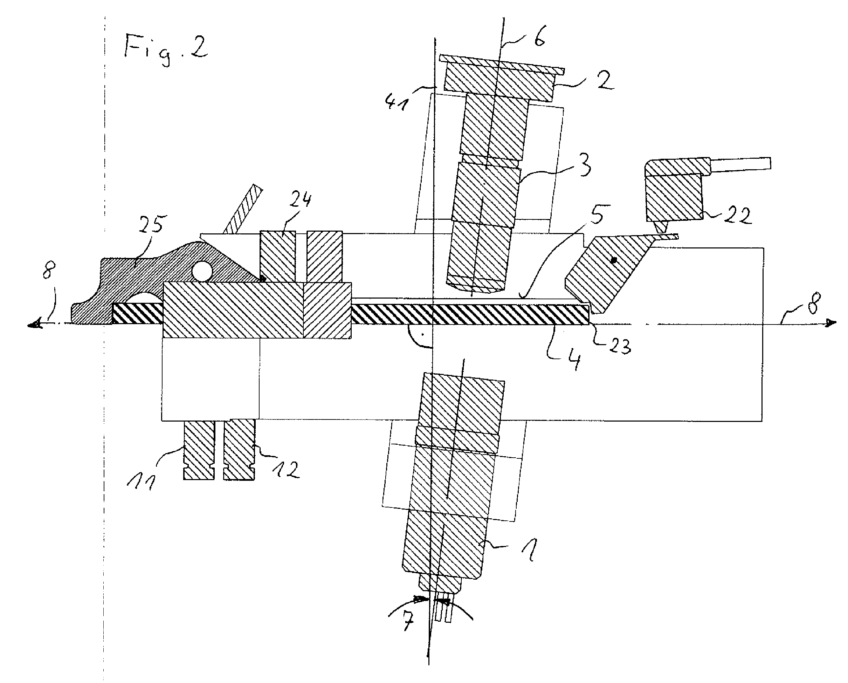

Wesentliche Elemente der Vorrichtung sind eine optische Einrichtung mit einer Lichtquelle 1, einem optischen Sensor in Form eines CCD-Sensors 2 und mit einer letzteren vorgeschalteten bildgebenden Optik 3. Diese optische Einrichtung wird im Bereich zwischen Lichtquelle 1 und Optik 3 durch einen Probenträger 4 durchsetzt, der in einer Aufnahme 5 der Vorrichtung eingesetzt ist und in dem die zu untersuchende Flüssigkeit befindlich ist. In Fig. 1 ist der Probenträger 4 aus Übersichtlichkeitsgründen um 90° um seine Längsmittelachse gedreht dargestellt. Er ist so angeordnet, dass das weiter unten noch im Einzelnen beschriebene Fenster des Probenträgers 4, durch welches die Flüssigkeit von zwei Seiten sichtbar ist, zwischen Lichtquelle 1 und Optik 3 des optischen Sensors 2 angeordnet ist, also durch die optische Achse 6 der optischen Einrichtung durchschnitten wird und zwar nicht senkrecht (die senkrechte Durchstrahlungsrichtung 41 ist in Fig. 2 dargestellt), sondern in einem Winkel 7 von hier 6°. Diese optische Einrichtung ist mittels eines Schrittmotors längs der Aufnahme 5 mit dem darin befindlichen Probenträger 4 verfahrbar, so dass mit jedem Verfahrschritt ein anderer Teil der zu untersuchenden Flüssigkeit in das Untersuchungsfeld der optischen Einrichtung gelangt. Die Verschieberichtung ist in Fig. 1 und 2 mit 8 gekennzeichnet.

Der Probenträger 4 weist einen Flüssigkeitseinlass 9 und einen Flüssigkeitsauslass 10 auf, die, wenn der Probenträger 4 in seiner bestimmungsgemäßen Position innerhalb der Aufnahme 5 angeordnet ist, mit einer Zuführleitung 11 bzw. mit einer Abführleitung 12 innerhalb der Vorrichtung verbunden sind. Nahe den Flüssigkeitsanschlüssen 9 und 10 ist innerhalb der Leitungen 11 und 12 jeweils ein Absperrventil 13, 14 angeordnet. Die Zuführleitung 11 schließt in dem dargestellten Ausführungsbeispiel gemäß Fig. 1 an eine Brauchwasserleitung 15 an. Die Zuführleitung ist über ein erstes Absperrventil 16 und ein Druckreduzierventil 17 und das Absperrventil 13 mit dem Flüssigkeitseinlass 9 verbunden. Im dargestellten Ausführungsbeispiel sind an die Leitung 11 über zwei Absperrventile 18 Probenbehälter 19 angeschlossen, die eine Probeentnahme mittels eines Behälters ermöglichen. Die Abführleitung 12 mündet in einer Abflussleitung 20.

Der optische Erfassungsvorgang einschließlich der schrittweisen Bewegung der optischen Einrichtung wird von einer Steuer- und Regeleinrichtung 21 gesteuert, die auch den Mikroprozessor umfasst, in dem die Auswertung erfolgt.

Da der Probenträger 4 innerhalb der Aufnahme 5 austauschbar angeordnet ist, das heißt werkzeugfrei entnommen und durch einen anderen Probenträger 4 ersetzt werden kann, weist die Vorrichtung einen ersten Schalter 22 auf, welcher von einem stirnseitigen Vorsprung 23, der am einführseitigen Ende des Probenträgers 4 außermittig angeordnet ist, geschaltet wird. Dieser Schalter 22 ist nur dann geschlossen, wenn der Probenträger 4 in seiner bestimmungsgemäßen Position in der Aufnahme 5 sitzt, in welcher Anschlüsse 9 und 10 mit den Leitungen 11 und 12 verbunden sind. Weiterhin ist ein zweiter Schalter 24 vorgesehen, der von einem schwenkbaren Riegel 25 geschaltet wird, und zwar wenn der Riegel 25 in der in Fig. 2 dargestellten Position befindlich ist, in welcher er den Probenträger 4 formschlüssig in der bestimmungsgemäßen Position in der Aufnahme 5 sichert. Diese Schalter 22 und 24 sind mit der Steuer- und Regeleinheit 21 verbunden und stellen sicher, dass die optische Erfassung der Probe nur dann erfolgt, wenn diese Schalter geschlossen sind, das heißt der Probenträger 4 in seiner bestimmungsgemäßen Position in der Aufnahme 5 angeordnet und durch den Riegel 25 in dieser Position gesichert ist. Die Steuer- und Regeleinheit 21 stellt mittels der Schalter 22 und 24 darüber hinaus sicher, dass die Ventile 13 und 14, mit denen die Zufuhr und Abfuhr von Flüssigkeit zum Probenträger 4 erfolgt, nur dann geöffnet werden können, wenn der Probenträger in seiner bestimmungsgemäßen Position ist und somit Anschlüsse 9 und 10 mit den entsprechenden Anschlüssen der Leitungen 11 und 12 innerhalb der Aufnahme 5 verbunden sind.

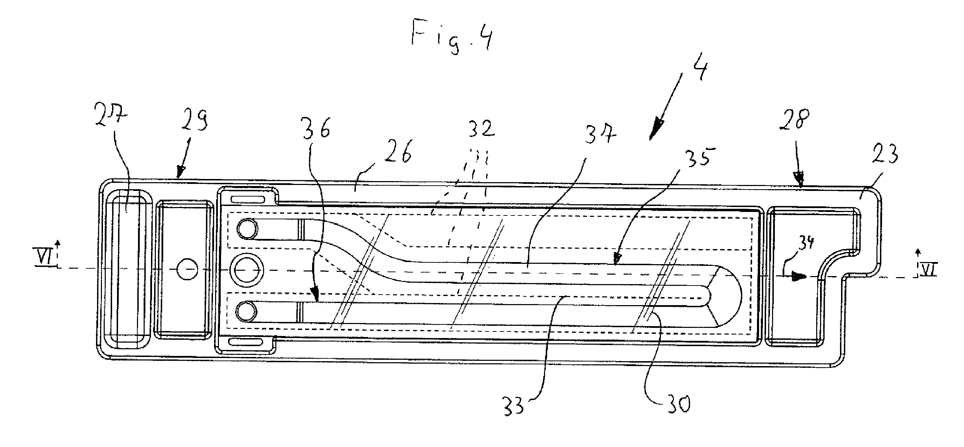

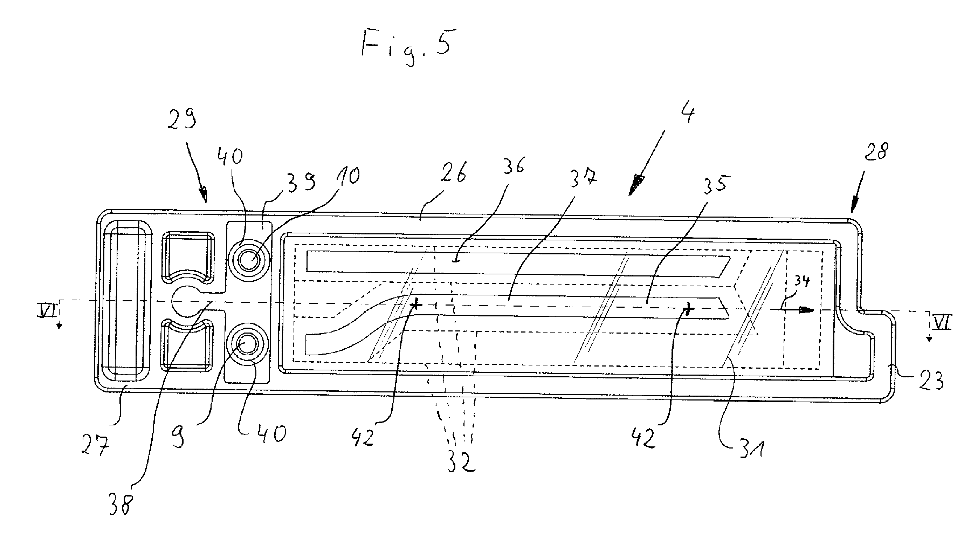

Der Probenträger 4 weist eine im Wesentlichen langgestreckte flache Quaderform auf und ist am einsteckseitigen Ende stirnseitig mit dem Vorsprung 23 versehen, der in eine entsprechende endseitige Ausnehmung in der Aufnahme 5 eingreift und dafür sorgt, dass der Probenträger 4 nur in der Stellung vollständig in die Aufnahme 5 eingeführt werden kann, in welcher der Vorsprung 23 von oben gesehen links der Längsmittellinie und in den Fig. 4 und 5 dargestellten Schnittlinie VI - VI liegt. Auf diesen Vorsprung 23 kann verzichtet werden, wenn der Querschnitt nicht wie beim Ausführungsbeispiel rechteckig sondern beispielsweise trapezförmig oder asymmetrisch dreieckig ausgebildet ist und die Aufnahme einen entsprechenden Querschnitt aufweist, so dass ein Einschieben nur in einer eindeutig vorgegebenen Stellung möglich ist, die, wenn der Probenträger 4 das Ende der Aufnahme 5 erreicht hat, die bestimmungsgemäße Position darstellt.

Der Probenträger 4 weist einen umlaufenden Rahmen 26 auf, der sowohl die Längsseiten als auch die Stirnseiten und den Vorsprung 23 sowie am anderen Ende ein Griffteil 27 bildet. Der Rahmen 26 ist als Kunststoffspritzgussteil ausgebildet und bestimmt die Außenkontur des Probenträgers 4. Der Rahmen 26 weist zwischen einem Endteil 28, welches den Vorsprung 23 aufweist, und einen Endteil 29, welches den Griffteil 27 aufweist, einen zu beiden Flachseiten des Probenträgers 4, also in der bestimmungsgemäßen Position zur Oberseite und zur Unterseite hin, eine Abstufung nach innen auf. Diese Abstufung bildet einen Rahmen für ein Fenster, welches durch den nach innen abgestuften Teil des Rahmens 26 und zwei an der Ober- bzw. Unterseite darin eingegliederte durchsichtige und aus Kunststoff bestehende Scheiben 30 und 31 gebildet ist, die durch Schweißen mit dem Rahmen 26 stoffschlüssig, unlösbar fest und dicht verbunden sind. Die Schweißnähte sind in den Figuren mit 32 gekennzeichnet. Die Abstufung des Rahmens 26 nach innen ist so gewählt, dass die obere Scheibe 30 und die untere Scheibe 31 zurückspringend im Rahmen 26 angeordnet sind, das heißt beim Einschieben des Probenträgers 4 in die Aufnahme 5 stets mit Abstand zur Aufnahme angeordnet sind und somit durch den Einschiebevorgang bzw. das Herausnehmen nicht belastet, insbesondere nicht zerkratzt werden können. Im vorliegenden Ausführungsbeispiel sind die Scheiben 30 und 31 durch Laserschweißen mit dem Rahmen 26 verbunden. Der Rahmen 26 ist somit lichtabsorbierend, hier schwarz. Alternativ kann eine solche Verbindung auch durch Ultraschallschweißen erfolgen, wenn eine Laserschweißung nicht möglich ist.

Der Rahmen 26 weist innerhalb des Fensters, also im Bereich zwischen den Scheiben 30 und 31, eine Längsrippe 33 auf, welche zwei verbundene Kanäle zwischen dem Flüssigkeitseinlass 9 und dem Flüssigkeitsauslass 10 bildet. Einlass 9 und Auslass 10 sind an der Unterseite des Probenträgers 4 angeordnet und in Einschubrichtung 34 nebeneinander angeordnet. Diese Längsrippe 33 endet mit Abstand zum Endteil 28, so dass dort eine Verbindung der beiden Kanäle gebildet ist. Hierdurch wird ein Kanal 37 gebildet, der, wie insbesondere in den Figuren 4 und 5 deutlich sichtbar ist, vom Flüssigkeitseinlass 9 zunächst ein Stück in Längs- und Einschubrichtung 34 des Probenträgers 4 verläuft, dann schräg zur Mitte in einen langgestreckten Kanalabschnitt 35 übergeht, nahe dem Endteil 28 um 180° geführt ist, um dann in einen langgestreckten Kanalabschnitt 36 überzugehen, der in Längsrichtung des Probenträgers gradlinig bis zum Flüssigkeitsauslass 10 verläuft. Dabei bilden die Rippe 33 und die Abstufungen an der anderen Kanalseite die rahmenseitige Begrenzung des Kanals 37, der im Übrigen durch die Scheiben 30 und 31 an der Ober- und Unterseite begrenzt ist. Dabei dient insbesondere der Kanalabschnitt 35, der in der Mitte des Probenträgers liegt, der optischen Einrichtung zur Erfassung von Partikeln innerhalb der Flüssigkeit, wohingegen der Kanalabschnitt 36 der Rückführung zum Flüssigkeitsauslass 10 dient. Wie anhand der Figuren 4 und 5 ersichtlich ist, sind die Scheiben 30 und 31 unmittelbar neben dem Kanal 37 mit der Längsrippe 33 bzw. mit dem abgestuften Bereich des Rahmens 26 verschweißt. Dies dient zur Erhöhung der Stabilität des Probenträgers einerseits und zum anderen um dem Kanal die erforderliche Druckfestigkeit zu geben, damit bei Druckbeaufschlagung des Kanals 37 die auf die Scheiben 30 und 31 wirkenden Kräfte zumindest teilweise vom Rahmen aufgenommen werden können.

Im Bereich des Flüssigkeitseinlasses 9 und des Flüssigkeitsauslasses 10 ist der Rahmen 26 an der Unterseite zurückspringend ausgebildet, so dass sich eine gegenüber der Außenkontur des Rahmens 26 abgesenkte Fläche ergibt, welche den Flüssigkeitseinlass 9 und den Flüssigkeitsauslass 10 umfasst sowie darüber hinaus einen stegförmigen Bereich 38. Diese abgesetzte Fläche ist mit einem weichelastischen Kunststoff ausgespritzt, wobei der stegförmige Bereich 38 im Wesentlichen einer sicheren Fixierung dient, wohingegen der Bereich um den Flüssigkeitseinlass 9 und den Flüssigkeitsauslass 10 als umlaufende und gegenüber der Kontur des Rahmens 26 nach unten vorspringende Dichtung ausgebildet ist. Dieses elastische im Ausführungsbeispiel aus Silikon bestehende Material bildet um die jeweiligen Ein- bzw. Auslässe 9, 10 umlaufende ringförmige Dichtungen 40, welche beim Einschieben des Probenträgers 4 in die Aufnahme 5 bei Erreichen der bestimmungsgemäßen Position den Einlass 9 und den Auslass 10 dicht mit entsprechenden Leitungsanschlüssen in der Aufnahme 5 verbinden und abdichten.

Zum Betrieb der Vorrichtung wird bei nach oben geschwenktem Riegel 25 ein Probenträger 4 in Richtung 34 in die Aufnahme 5 der Vorrichtung eingeschoben bis der Vorsprung 23 in der endseitigen Ausnehmung innerhalb der Aufnahme 5 liegt, so dann wird der Riegel 25 nach unten geschwenkt und der Probenträger 4 formschlüssig in der Aufnahme 5 gesichert. In dieser Stellung wird der erste Schalter 22 durch den Vorsprung 23 geschlossen, der zweiten Schalter 24 wird durch den heruntergeschwenkten Riegel geschlossen. Dann ist sichergestellt, dass der Probenträger 4 in seiner bestimmungsgemäßen Position in der Aufnahme 5 ist, in welcher der Flüssigkeitseinlass 9 und der Flüssigkeitsauslass 10 an der Unterseite des Probenträgers 4 mit den entsprechenden Leitungen 11 und 12 in der Aufnahme 5 fluchten und über die Ringdichtungen 40 dichtend mit diesen Leitungen verbunden sind. Über die Steuerund Regeleinheit 21 erfolgt nunmehr die Freigabe der Vorrichtung, das heißt es werden das Ventil 16 und die Ventile 13 und 14 öffnend angesteuert, so dass das zu untersuchende Brauchwasser über die Brauchwasserleitung 15 in die Vorrichtung einströmt, wobei gegebenenfalls durch Öffnen eines der Absperrventile 18 eine Probe in einen Behälter 19 abgezapft werden kann, wenn beispielsweise festgestellt worden ist, dass der Bakteriengehalt der untersuchten Flüssigkeit zu hoch ist. Durch das Druckreduzierventil 17 wird der Druck der Brauchwasserleitung 15, der beispielsweise sieben bar beträgt, auf zwei bar reduziert. Das Brauchwasser gelangt über das Ventil 13 und die Leitung 11 zum Flüssigkeitseinlass 9 des Probenträgers 4, strömt dort durch den Kanal 37 bis zum Flüssigkeitsauslass 10 und von dort in die Abführleitung 12 durch das Absperrventil 14 zur Abflussleitung 20. Zur Partikelerfassung wird nun zunächst das Ventil 14 geschlossen, bis sich im Gesamtsystem, das heißt auch im Kanal 37 des Probenträgers 4, ein Druck von beispielsweise zwei bar aufgebaut hat. Sodann wird das Absperrventil 13 verschlossen und eine Verweilzeit gewartet, bis sich die Flüssigkeit im Kanal 37 und insbesondere die darin befindlichen Partikel beruhigt haben, also quasi stationär sind. Dann wird mit der optischen Einrichtung ein Abschnitt des Kanals 37, mittels des optischen Sensors 2 erfasst und mittels der Steuerund Regeleinheit 21 hinsichtlich der darin befindlichen Partikel ausgewertet. Sodann wird die optische Einrichtung mittels des Schrittmotors einen Schritt in Verschieberichtung 8 verschoben, wonach ein weiterer optischer Erfassungsvorgang (Scan) erfolgt und dies so lange, bis die gewünschte Anzahl von Scannvorgängen über die Länge des Kanals 37 erfolgt sind. Nachfolgend wird die optische Einrichtung zurückgefahren und es werden die Ventile 13 und 14 geöffnet um die im Probenträger befindliche Flüssigkeit auszutauschen.

Auf diese Weise können eine Vielzahl von Flüssigkeitsproben hinsichtlich der darin befindlichen Partikel sowohl qualitativ als auch quantitativ erfasst werden, das heißt es kann mit der Auswertelektronik in der Steuer- und Regeleinheit 21 zum einen festgestellt werden, um was für Partikel es sich handelt, beispielweise Bakterien oder anorganische Verunreinigungen. Zum andere kann ermittelt werden, in welcher Menge diese vorhanden sind.

Wenn, was ebenfalls automatisiert erfolgen kann, mittels der optischen Einrichtung festgestellt wird, dass sich im Probenträger derart viele Ablagerungen gebildet haben, sei es organischer Belag oder andere Partikel, dass die Funktion beeinträchtigt ist, kann dies durch eine Anzeige an der Vorrichtung angezeigt werden. Dann muss der Probenträger 4 durch einen anderen Probenträger ersetzt werden, indem der Riegel 25 geöffnet, der in der Aufnahme 5 befindliche Probenträger 4 herausgezogen und durch einen anderen ersetzt wird.

Im vorstehenden Ausführungsbeispiel ist eine Mehrfachnutzung ein und desselben Probenträgers 4 vorgesehen, dadurch, dass die Flüssigkeit in dem Probenträger ausgetauscht wird. Es kann auch, wenn im Flüssigkeitseinlass 9 und dem Flüssigkeitsauslass 10 entsprechende Rückschlagklappen vorgesehen sind, der Probenträger 4 zur einmaligen Nutzung ausgelegt sein, so dass mit Entnahme des Probenträgers auch die darin untersuchte Flüssigkeit entnommen wird und innerhalb des Probenträgers 4 als quasi Probenbehälter gelagert werden kann.

Innerhalb des Probenträgers 4, und zwar im Bereich des Fensters, und zwar dort, wo der geschlossene Kanal 37 zur optischen Erfassung der Flüssigkeit vorgesehen ist, also im Bereich des Kanalabschnittes 35, sind zwei Markierungen 42 vorgesehen, und zwar an der Innenseite der oberen Scheibe 30, die dem Sensor 2 näher ist als die untere Scheibe 31. Diese Markierungen kennzeichnen hier den Bereich der Messstrecke, d. h. innerhalb der die optische Einrichtung den Probenträger 4 abfährt, um die im Kanalabschnitt 35 befindliche Flüssigkeit zu untersuchen. Dabei dienen die Markierungen 42 sowohl zur Kalibrierung des optischen Systems als auch für den Autofokus des optischen Systems, um auf die Ebene zu fokussieren, in welcher sich die zu untersuchende Flüssigkeit befindet.

### Bezugszeichenliste

- 1: Lichtquelle
- 2: Optischer Sensor
- 3: Optik
- 4: Probenträger
- 5: Aufnahme
- 6: Optische Achse
- 7: Winkel
- 8: Verschieberichtung
- 9: Flüssigkeitseinlass
- 10: Flüssigkeitsauslass
- 11: Zuführleitung
- 12: Abführleitung
- 13: Absperrventil
- 14: Absperrventil
- 15: Brauchwasserleitung
- 16: Absperrventil
- 17: Druckreduzierventil
- 18: Absperrventil
- 19: Probenbehälter
- 20: Abflussleitung
- 21: Steuer- und Regeleinheit
- 22: Erster Schalter
- 23: Vorsprung
- 24: Zweiter Schalter
- 25: Riegel
- 26: Rahmen
- 27: Griffteil
- 28: Endteil
- 29: Endteil
- 30: Obere Scheibe
- 31: Untere Scheiben
- 32: Schweißnähte
- 33: Längsrippe
- 34: Einschubrichtung
- 35: Kanalabschnitt
- 36: Kanalabschnitt
- 37: Kanal
- 38: Stegförmiger Bereich
- 39: Elastisches Material
- 40: Ringdichtung
- 41: Senkrechte Durchstrahlungsrichtung
- 42: Markierung

## Patentansprüche

1. Vorrichtung zum qualitativen und/oder quantitativen Erfassen von Partikeln in einer Flüssigkeit, mit einer Lichtquelle (1), einem optischen Sensor (2) und einem dazwischen angeordneten Probenträger (4) zur Aufnahme von zu untersuchender Flüssigkeit, wobei der Probenträger (4) relativ zumindest zum Sensor (2) bewegbar ist und über einen Flüssigkeitseinlass (9) mit einer Leitung (11) zum Zuführen von Flüssigkeit und über einen Flüssigkeitsauslass (10) mit einer Leitung (12) zum Abführen von Flüssigkeit verbindbar ist, **dadurch gekennzeichnet, dass** der Probenträger (4) austauschbar in einer Aufnahme (5) der Vorrichtung angeordnet ist.

2. Vorrichtung nach Anspruch 1, **dadurch gekennzeichnet, dass** zwischen den Leitungsanschlüssen (9, 10) im Probenträger (4) ein geschlossener Kanal (37) gebildet ist, der zumindest abschnittsweise an zwei vorzugsweise gegenüberliegenden Seiten eine durchsichtige Wandung (30, 31) aufweist.

3. Vorrichtung nach Anspruch 1 oder 2, **dadurch gekennzeichnet, dass** Mittel zum Absperren (13, 14) mindestens einer, vorzugsweise beider Leitungen (11, 12) zum Zu- und Abführen von Flüssigkeit vorgesehen sind.

4. Vorrichtung nach einem der vorhergehenden Ansprüche, **dadurch gekennzeichnet, dass** beide Leitungen (11, 12) von unten in den Probenträger (4) münden.

5. Vorrichtung nach einem der vorhergehenden Ansprüche, **dadurch gekennzeichnet, dass** die Lichtquelle und der vorzugsweise oberhalb des Probenträgers angeordnete optische Sensor innerhalb der Vorrichtung bevorzugt schrittweise bewegbar zum Probenträger angeordnet sind.

6. Vorrichtung nach einem der vorhergehenden Ansprüche, **dadurch gekennzeichnet, dass** eine zwischen Lichtquelle (1) und optischem Sensor (2) und ggf. weiteren optischen Komponenten (3) gebildete optische Achse (6) in einem Winkel (7) zwischen 4° bis 8° zur senkrechten Durchstrahlungsrichtung (41) durch den Probenträger (4) angeordnet ist.

7. Vorrichtung nach einem der vorhergehenden Ansprüche, **dadurch gekennzeichnet, dass** Mittel (22, 24) zwischen Aufnahme (5) und Probenträger (4) vorgesehen sind, welche eine Leitungsverbindung zu dem Flüssigkeitseinlass (9) und/oder zu dem Flüssigkeitsauslass (10) nur zulassen, wenn der Probenträger (4) in seiner bestimmungsgemäßen Position in der Aufnahme (5) angeordnet ist.

8. Vorrichtung nach Anspruch 7, **dadurch gekennzeichnet, dass** die Mittel einen ersten Schalter (22) aufweisen, welcher beim Erreichen der bestimmungsgemäßen Position des Probenträgers (4) in der Aufnahme (5) schaltet.

9. Vorrichtung nach einem der vorhergehenden Ansprüche, **dadurch gekennzeichnet, dass** Formschlussmittel (23) zwischen Aufnahme (5) und Probenträger (4) vorgesehen sind, welche nur eine bestimmungsgemäße Anordnung des Probenträgers (4) in der Aufnahme (5) sicherstellen.

10. Vorrichtung nach einem der vorhergehenden Ansprüche, **dadurch gekennzeichnet, dass** ein vorzugsweise schwenkbarer Riegel (25)vorgesehen ist, mit welchem der Probenträger (4) in seiner bestimmungsgemäßen Anordnung in der Aufnahme (5) formschlüssig festlegbar ist.

11. Vorrichtung nach Anspruch 10, **dadurch gekennzeichnet, dass** die Mittel einen zweiten Schalter (24) aufweisen, welcher schaltet, wenn der Riegel (25) in der den Probenträger (4) in der Aufnahme (5) verriegelnden Stellung ist.

12. Probenträger für eine Vorrichtung nach einem der vorhergehenden Ansprüche, **dadurch gekennzeichnet, dass** er einen Tragrahmen (26) aufweist, der an zwei Seiten, vorzugsweise der Ober- und der Unterseite, jeweils mit einer durchsichtigen Platte (30, 31) abgeschlossen ist.

13. Probenträger nach Anspruch 12, **dadurch gekennzeichnet, dass** mindestens eine, vorzugsweise beide durchsichtigen Platten (30, 31) gegenüber einem umfänglich umlaufenden Rahmenteil zurückspringend angeordnet sind.

14. Probenträger nach Anspruch 8 oder 9, **dadurch gekennzeichnet, dass** ein Flüssigkeitseinlass (9) und ein Flüssigkeitsauslass (10) an der Unterseite, vorzugsweise in Einschubrichtung (34) nebeneinander angeordnet sind.

15. Probenträger nach einem der vorhergehenden Ansprüche, **dadurch gekennzeichnet, dass** zwischen dem Flüssigkeitseinlass (9) und dem Flüssigkeitsauslass (10) mindestens eine kanalbildende Rippe (33) vorgesehen ist, die vorzugsweise Teil des Rahmens (26) bildet.

16. Probenträger nach einem der vorhergehenden Ansprüche, **dadurch gekennzeichnet, dass** er an der Einsteckseite einen Vorsprung (23) aufweist, der außermittig angeordnet und ein Formschlussmittel zum Positionieren bildet.

17. Probenträger nach einem der vorhergehenden Ansprüche, **dadurch gekennzeichnet, dass** er ein den Rahmen (26) bildenden Kunststoffspritzgussteil aufweist, das mit den durchsichtigen Platten (30, 31) stoffschlüssig, vorzugsweise durch Schweißen fest und dicht verbunden ist.

18. Probenträger nach einem der vorhergehenden Ansprüche, **dadurch gekennzeichnet, dass** er im Bereich der Ein- und Auslässe (9, 10) eine Ausnehmung in dieser Seite des Rahmenteils aufweist, welche mit einem elastischen und dichtungsbildenden Kunststoff (39) ausgefüllt ist.

19. Probenträger nach einem der vorhergehenden Ansprüche, **dadurch gekennzeichnet, dass** an einer Seite, vorzugsweise einer Schmalseite ein Griffstück (27) vorgesehen ist, welches Teil des Rahmens (26) bildet.

20. Probenträger nach einem der vorhergehenden Ansprüche, **dadurch gekennzeichnet, dass** er eine im wesentlichen langgestreckte und flache Quaderform aufweist, wobei die Flachseiten mit den durchsichtigen Platten (30, 31) versehen sind und die langgestreckten Seiten in Einschubrichtung (34) angeordnet sind.

21. Probenträger nach einem der vorhergehenden Ansprüche, **dadurch gekennzeichnet, dass** der geschlossene Kanal (37) innenseitig, zumindest an der Innenseite der durchsichtigen Wandungen (30, 31) mit einer Beschichtung und/oder einer Oberflächenstrukturierung versehen ist, welche die Bildung von Ablagerungen vermindert.

22. Probenträger nach einem der vorhergehenden Ansprüche, **dadurch gekennzeichnet, dass** in dem Bereich, der zur optischen Erfassung der Flüssigkeit vorgesehen ist, mindestens eine Markierung (42) zwischen den durchsichtigen Wandungen (30, 31) vorgesehen ist, vorzugsweise an einer Innenseite einer Wandung (30, 31)

23. Probenträger nach einem der vorhergehenden Ansprüche, **dadurch gekennzeichnet, dass** der geschlossene Kanal (37) in dem Bereich, der zur optischen Erfassung der Flüssigkeit vorgesehen ist, einen Kanalquerschnitt aufweist, dessen Breite größer als dessen Höhe in senkrechter Durchstrahlungsrichtung (41) ist, vorzugsweise 1,5 bis 3 mal so groß ist.

24. Verfahren zum Erfassen von Partikeln in einer Flüssigkeit mit einer Vorrichtung nach einem der vorhergehenden Ansprüche, **dadurch gekennzeichnet, dass** ein Probenträger (4) nach einem der vorhergehenden Ansprüche in seine bestimmungsgemäße Position in der Vorrichtung verbracht wird, wonach in einem ersten Schritt über die Zuführleitung (11) die zu untersuchende Flüssigkeit in den Probenträger (4) eingeleitet wird, wonach in einem zweiten Schritt die Abführleitung (12) abgesperrt wird und nach Druckaufbau innerhalb des Probenträgers (4) in einem dritten Schritt die Zuführleitung (11) verschlossen wir, wonach eine Verweilzeit lang gewartet wird und in einem vierten Schritt die optische Erfassung der Probe erfolgt.

25. Verfahren nach Anspruch 24, **dadurch gekennzeichnet, dass** nach dem optischen Erfassen in einem fünften Schritt die Zuführ- und Abführleitungen (11, 12) geöffnet werden, wonach die im Probenträger (4) befindliche Flüssigkeit ersetzt wird und der Zyklus mit dem zweiten Schritt beginnend wiederholt wird.
